# EUROPEAN PATENT APPLICATION

(11) **EP 4 706 545 A1**
(43) Date of publication of application: **11.03.2026**
(21) Application number: 24815191.2
(22) Date of filing: 14.05.2024
(51) Int. Cl.: A61B 8/00

(54) **GEL SHEET, ULTRASONIC INSPECTION SYSTEM, AND METHOD FOR MANUFACTURING GEL SHEET**

(30) Priority: 29.05.2023 JP 2023087978
(71) Applicant: Salmontech Inc., Kumamoto-shi, Kumamoto 860-0862 (JP)
(72) Inventor: TANABE, Masayuki, Kumamoto-shi, Kumamoto 860-0862 (JP)
(74) Representative: Isarpatent
(86) International application number: PCT/JP2024/017730
(87) International publication number: WO 2024/247707

(57) **Abstract**

An object is to provide a gel sheet, an ultrasonic inspection system, and a method for manufacturing a gel sheet that enables positioning and setting of an ultrasonic probe to be performed easily. A gel sheet 20 used in an ultrasonic inspection system 1 includes, in a predetermined region of a front surface of sheet form, a formed portion 21 differing in movement resistance in comparison to other regions. The formed portion 21 has a plurality of projections 211 and recesses 212 formed periodically at a predetermined uniform width in correspondence to a scanning position of the ultrasonic probe 12 and functions as a scale. During measurement, by counting the number of scale marks from a starting point O of the projections 211, a measurement site that is specific to each subject M can be ascertained as a scale mark position and therefore, a user can easily identify the scanning position of the ultrasonic probe 12 without checking his/her hand.

## Description

### Technical Field

The present invention relates to a gel sheet, an ultrasonic inspection system, and method for manufacturing a gel sheet.

### Related Art

Biological inspection using ultrasonic images has been conventionally put to practical use in medical fields and non-medical fields. An ultrasonic image is obtained by irradiating ultrasonic waves from an ultrasonic probe onto a prescribed portion of a living body of a subject and electrically detecting echo signals from the living body in an ultrasonic inspection device connected to the ultrasonic probe via a connector. As a method of driving the ultrasonic probe, there is known, for example, an electronic scanning method with which a plurality of ultrasonic vibrators (ultrasonic transducers) that transmit and receive ultrasonic waves are arranged and the ultrasonic vibrator to be driven is selectively switched by an electronic switch, etc.

Also, in recent years, an ultrasonic probe of a wireless type that is connected by wireless communication to an ultrasonic inspection device has been developed as disclosed for example in Patent Literature 1. Such an ultrasonic probe of the wireless type transmits received signals output from a vibrator array to the ultrasonic inspection device side by wireless communication or incorporates a circuit for signal processing and upon digitally processing the received signals output from the vibrator array, performs transmission by wireless communication to the ultrasonic inspection device. Based on the signals thus transmitted wirelessly from the ultrasonic probe, the ultrasonic inspection device generates and displays ultrasonic images.

### Citation List

### Patent Literature

Patent Literature 1: Japanese Patent Application Publication No. 2013-215553

### SUMMARY OF INVENTION

### Problem to Be Solved by Invention

Incidentally, when using an ultrasonic inspection device to perform a follow-up observation of a specific part of a subject to be measured, a user at times measures the same location as a measurement location measured previously. In such as case, the user sets the ultrasonic probe in a state where a gel sheet that transmits ultrasonic waves is placed at a predetermined position of a body portion of the subject and determines the measurement site while viewing an image displayed on a display screen of the ultrasonic inspection device.

In regard to identification of the measurement site, although, for example, a doctor, a clinical laboratory technician, or a nurse who is accustomed to operation can identify the measurement site comparatively easily, for a user who is unaccustomed to operation of the ultrasonic inspection device, identification of the same measurement position as in the previous measurement is not easy. Also, even for a doctor, a clinical laboratory technician, or a nurse who is accustomed to operation of the ultrasonic inspection device, it is desirable that the ultrasonic inspection device can be operated more easily.

Also, in cases where an inspection is performed with the ultrasonic probe being as it is in its initial setting, the setting of the ultrasonic inspection device is at times unsuitable and a desired display screen is not obtained. In this case, adjustment, for example, of gain, focus, or depth must be set according to each subject. Even such adjustment requires extremely advanced skills for a user unaccustomed to the operation of the ultrasonic inspection device.

The present invention has been made in view of the above points and an object thereof is to provide a gel sheet, an ultrasonic inspection system, and a method for manufacturing a gel sheet that enables positioning and setting of an ultrasonic probe to be performed easily.

### Means for Solving Problem

To achieve the above object, a gel sheet of the present invention is a gel sheet used by being interposed between an ultrasonic probe and a subject and where a formed portion to which a treatment including any one of a projection, a recess, a periodically formed unevenness portion, or a predetermined surface treatment is applied in correspondence to predetermined information is included in a predetermined region of a front surface of the gel sheet and the region in which the formed portion is formed differs in movement resistance against the ultrasonic probe in comparison to other regions.

Here, by the formed portion that is formed in correspondence to the predetermined information being included in the predetermined region of the front surface of the gel sheet, for example, information on a measurement position of the ultrasonic probe, personal information on the subject, and other predetermined information can be included in the formed portion.

Also, by the region in which the formed portion is formed being different in movement resistance against the ultrasonic probe in comparison to the other regions, a user can ascertain the position of the formed portion based on sensation without having to check the position of the formed portion by watching his/her hand.

Also, by the formed portion having the treatment including any one of a projection, a recess, a periodically formed unevenness portion, or a predetermined surface treatment applied thereto, the user can reliably ascertain the position of the formed portion based on sensation by the formed portion to which such surface treatments have been applied.

Also, when the formed portion is formed in a region corresponding to a scanning location of the ultrasonic probe, the user can ascertain the position of the measurement position of the ultrasonic probe based on sensation without checking his/her hand.

Also, when the formed portion is formed in correspondence to the predetermined information related to the subject, specific information related to the subject, for example, a height, weight, age, sex, past measurement result, etc., of the subject can be registered in the formed portion. Then, by moving the ultrasonic probe over the formed portion, the information corresponding to the shape of the formed portion can be converted to digital information in an external device and used.

Also, when the formed portion is formed in correspondence to a setting condition of the ultrasonic probe to be applied to the subject, information corresponding to the shape of the formed portion can be converted to digital information in an external device and the ultrasonic probe can be set based on the converted digital information. Therefore, the user does not need to manually adjust the setting condition of the ultrasonic probe in accordance with the subject and can set the ultrasonic probe by scanning the ultrasonic probe over the formed portion.

Also, when the formed portion has a non-transmitting region that does not transmit the ultrasonic waves emitted from the ultrasonic probe and the non-transmitting region is an air gap that is formed between the ultrasonic probe and the front surface of the gel sheet, the non-transmitting region that does not transmit the ultrasonic waves and a transmitting region that transmits the ultrasonic waves are formed continuously as the formed portion. And, for example, by defining, as digital information, a case where the ultrasonic waves are not transmitted as "0" and a case where the ultrasonic waves are transmitted as "1" respectively, various information can be registered in the formed portion as digital information combining "0"s and "1"s according to the shape of the formed portion.

Also, when the formed portion is formed periodically in a rectilinear shape, a matrix shape, or an arcuate shape formed along a displacement direction of the ultrasonic probe in plan view of the gel sheet, the user can easily ascertain a range of measurement positions of the subject by moving the ultrasonic probe along the shape of the unevenness portion.

To achieve the above object, an ultrasonic inspection system includes an ultrasonic inspection device that has an ultrasonic probe having a sensor portion detecting reflected waves of emitted ultrasonic waves reflected from a subject and an image converting portion converting an electric signal from the ultrasonic probe to ultrasonic image data, a gel sheet that is used by being interposed between the ultrasonic probe and the subject and has, in a predetermined region of a front surface, a formed portion to which a treatment including any one of a projection, a recess, a periodically formed unevenness portion, or a predetermined surface treatment is applied in correspondence to predetermined information, with the region in which the formed portion is formed being different in movement resistance against the ultrasonic probe in comparison to other regions, and a management server that has a storage portion storing the predetermined information corresponding to the formed portion and a collating portion collating, by aligning the ultrasonic probe with the formed portion, the information stored in the storage portion and the information corresponding to the formed portion and is capable of selecting, based on a collation result of the collating portion, desired information from the information stored in the storage portion.

Here, by including the ultrasonic probe that has the sensor portion detecting the reflected waves of the emitted ultrasonic waves reflected from the subject and the ultrasonic inspection device that has the image converting portion converting the electric signal from the ultrasonic probe to ultrasonic image data, measurement data measured by the ultrasonic probe can be converted to image data by the image converting portion to output a measurement result as an image.

Also, by including the gel sheet that is used by being interposed between the ultrasonic probe and the subject and has, in the predetermined region of the front surface, the formed portion to which a treatment including any one of a projection, a recess, a periodically formed unevenness portion, or a predetermined surface treatment is applied in correspondence to predetermined information, with the region in which the formed portion is formed being different in movement resistance against the ultrasonic probe in comparison to the other regions, for example, information on a measurement position of the ultrasonic probe, personal information on the subject, and other predetermined information can be included in the formed portion and a user can ascertain the position of the formed portion based on sensation without having to check the position of the formed portion by watching his/her hand.

Also, by including the management server that has a storage portion storing the predetermined information corresponding to the formed portion and the collating portion collating, by aligning the ultrasonic probe with the formed portion, the information stored in the storage portion and the information corresponding to the formed portion and is capable of selecting, based on a collation result of the collating portion, desired information from the information stored in the storage portion, the information corresponding to the formed portion can be read out from the storage portion by aligning the ultrasonic probe with the formed portion. Therefore, by registering specific information in the formed portion, the information registered in each formed portion can be checked in each measurement.

Also, when the information corresponding to the formed portion is a setting condition of the ultrasonic probe to be applied according to each subject, by aligning the ultrasonic probe with the formed portion during measurement of the subject and reading out the setting condition stored in the storage portion and corresponding to the information of the formed portion, the ultrasonic probe can be set automatically.

Also, when the information corresponding to the formed portion is specific information of the subject including a height, weight, sex, age, medical history, and inspection history of each subject, by aligning the ultrasonic probe with the formed portion during measurement of the subject and thereby reading out the specific information stored in the storage portion and corresponding to the information of the formed portion, the specific information of the subject can be checked and measurement of the subject can thus be performed safely and quickly.

To achieve the above object, a method for manufacturing a gel sheet of the present invention includes a step of marking a mark indicating a positional relationship of an ultrasonic probe and a gel sheet on the gel sheet in a state in which the gel sheet is interposed between the ultrasonic probe and a subject and a step of forming, in a predetermined region that includes a position corresponding to the mark marked on the gel sheet, a formed portion differing in movement resistance against the ultrasonic probe in comparison to other regions.

Here, by including the step of marking the mark indicating the positional relationship of the ultrasonic probe and the gel sheet on the gel sheet in the state in which the gel sheet is interposed between the ultrasonic probe and the subject, a measurement position of the ultrasonic probe that is in accordance with the subject can be marked on the gel sheet.

Also, by including the step of forming, in the predetermined region that includes the position corresponding to the mark marked on the gel sheet, the formed portion differing in movement resistance against the ultrasonic probe in comparison to the other regions, the formed portion can be formed in the region corresponding to the measurement position of the ultrasonic probe. Also, since the formed portion differs from the other regions in movement resistance against the ultrasonic probe, a user can ascertain the position of the ultrasonic probe without checking his/her hand during use.

### Effects of Invention

The gel sheet, the ultrasonic inspection system, and the method for manufacturing the gel sheet according to the present invention enable positioning and setting of the ultrasonic probe to be performed easily.

### BRIEF DESCRIPTION OF DRAWINGS

[FIG. 1] is an overall view of an ultrasonic inspection system according to a first embodiment.
[FIG. 2] is an external view of a gel sheet according to the first embodiment.
[FIG. 3] is a sectional view taken along line A-A of a formed portion of the gel sheet according to the first embodiment.
[FIGS. 4a to 4c] is a diagram showing another mode of the formed portion of the gel sheet according to the first embodiment.
[FIG. 5] is a diagram showing a manufacturing procedure of the gel sheet according to the first embodiment.
[FIG. 6] is an overall view of an ultrasonic inspection system according to a second embodiment.
[FIGS. 7a and 7b] shows external views of a gel sheet according to the second embodiment with FIG. 7(a) being a plan view and FIG. 7(b) being a sectional view taken along line B-B.

### DESCRIPTION OF EMBODIMENTS

Embodiments related to a gel sheet, an ultrasonic inspection system, and a method for manufacturing a gel sheet shall be described below with reference to the drawings to facilitate understanding of the present invention.

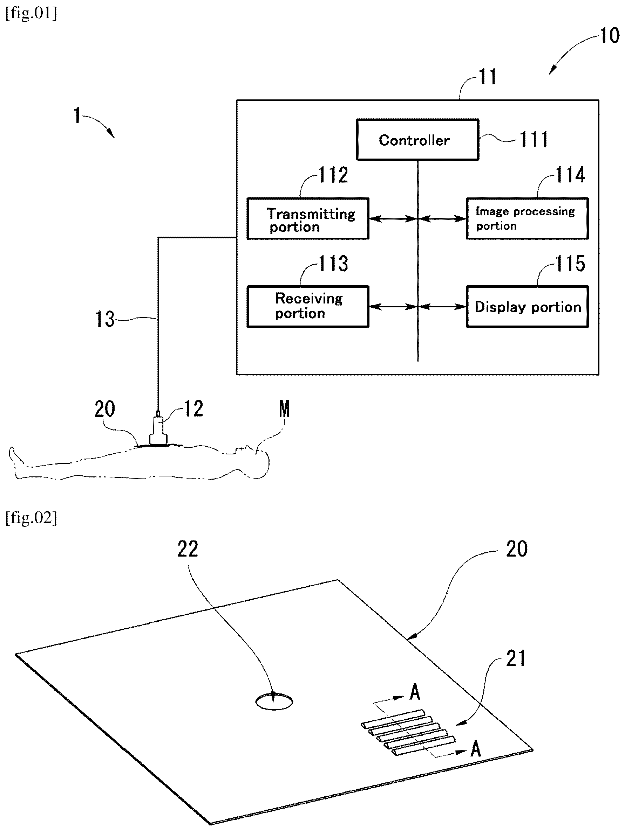

First, an outline of an ultrasonic inspection system 1 according to a first embodiment shall be described using FIG. 1. The ultrasonic inspection system 1 according to the first embodiment is a system that can be used to visualize and inspect a shape, properties, or dynamics, etc., of an interior of a living body of a subject M (for example, a human body) as an ultrasonic image and is constituted of an ultrasonic inspection device 10 and a gel sheet 20.

### [Ultrasonic inspection device]

As shown in FIG. 1, the ultrasonic inspection device 10 includes an ultrasonic inspection device main body 11 and an ultrasonic probe 12 and has an arrangement where the ultrasonic inspection device main body 11 and the ultrasonic probe 12 are connected via a cable 13. It can also be used, for example, with an ultrasonic probe that includes an ultrasonic inspection device function. In this case, for example, a smartphone can be used as a display portion 115.

The ultrasonic probe 12 can function as an acoustic sensor that transmits an ultrasonic beam (for example of approximately 1 to 30 MHz) into the living body of the subject M and receives an ultrasonic echo of the portion of the transmitted ultrasonic beam reflected inside the living body of the subject M and converts it to an electric signal and is constituted, for example, of a plurality of piezoelectric elements installed in an array or a matrix and a switching portion (not shown) for switchingly controlling the ON/OFF of drive states of the plurality of piezoelectric elements individually or in block units.

The piezoelectric elements of the ultrasonic probe 12 generate the ultrasonic beam based on drive signals (voltage pulses) supplied from the ultrasonic inspection device main body 11. The ultrasonic beam is thereby transmitted from the ultrasonic probe 12 to the subject M. When the ultrasonic beam is transmitted from the ultrasonic probe 12 to the subject M, the transmitted ultrasonic beam is reflected successively by discontinuity surfaces of acoustic impedance inside the living body of the subject M and the ultrasonic echo is received as a reflected wave signal by the piezoelectric elements. Ultrasonic inspection of ascertaining the state of the interior of the living body of the subject M can thereby be performed.

Although here, the ultrasonic probe 12 is that which transmits the ultrasonic beam into the living body of the subject M from an outer surface of the subject M and receives the ultrasonic echo, the ultrasonic probe 12 may be that which is used by insertion into an interior, etc., of a digestive tract, a blood vessel, etc., instead. Also, as the ultrasonic probe 12, any of a convex probe, a linear probe, a sector probe, or a two-dimensional probe, etc., can be applied.

The ultrasonic inspection device main body 11 is a device that generates the ultrasonic image based on the ultrasonic echo received by the ultrasonic probe 12 and is constituted of a controller 111, a transmitting portion 112, a receiving portion 113, an image processing portion 114, and the display portion 115.

First, the controller 111 performs overall control of the ultrasonic inspection device main body 11 and has a CPU as a computing device and a ROM and a RAM, etc., as storage devices. A basic program and basic setting data are stored in the ROM. The CPU reads a program in accordance with processing contents from the ROM, expands the program in the RAM, and executes the expanded program to control the ultrasonic inspection device main body 11.

The transmitting portion 112 is a transmitter transmitting a voltage pulse that is a drive signal to the ultrasonic probe 12 in accordance with an instruction of the controller 111. The transmitting portion 112 is constituted, for example, of a high-frequency pulse oscillator and a pulse setting portion, etc., adjusts the voltage pulse generated by the high-frequency pulse oscillator to be of a predetermined voltage amplitude, pulse width, and sending timing, and sends it to the ultrasonic probe 12,

The ultrasonic probe 12 has a plurality of channels and the transmitting portion 112 is capable of setting the voltage amplitude, the pulse width, and the sending timing of the voltage pulse for each channel. By setting appropriate delay times for the plurality of channels, the transmitting portion 112 can change a depth to be targeted or generate different pulse waveforms.

The receiving portion 113 is a receiver that receives, in accordance with an instruction of the controller 111, a received signal related to the ultrasonic echo generated by the ultrasonic probe 12. The receiving portion 113 is constituted, for example, of a preamp, an AD converting portion, and a receiving beamformer, etc.

The receiving portion 113 amplifies, according to each channel, the received signal related to the ultrasonic echo by the preamp and converts the signal to a digital signal. The receiving portion 113 can combine the received signals of the plurality of channels into one by performing phasing processing on the received signals of the respective channels.

The image processing portion 114 has a function of acquiring the received signal from the receiving portion 113 and generating the ultrasonic image of the interior of the living body of the subject M. For example, when the ultrasonic probe 12 transmits the ultrasonic beam in a depth direction, a signal intensity of the ultrasonic echo detected thereafter is accumulated continuously over time in a line memory by the image processing portion 114.

As the ultrasonic beam from the ultrasonic probe 12 scans the interior of the living body of the subject M, the image processing portion 114 sequentially accumulates the signal intensity of the ultrasonic echo at each scanning position in a line memory and generates two-dimensional data in frame units. The image processing portion 114 converts the signal intensities of the generated two-dimensional data to luminance values to generate the ultrasonic image that represents a two-dimensional structure inside a cross section including a transmission direction of the ultrasonic waves and a scanning direction of the ultrasonic waves.

When the ultrasonic image is generated, the image processing portion 114 further generates a display image that includes a display region of the ultrasonic image. The image processing portion 114 then sends the data of the generated display image to the display portion 115 and displays the image on the display portion 115. The image processing portion 114 sequentially renews the display image each time a new ultrasonic image is generated and makes the display portion 115 display the display image in the form of a moving image, a still image, etc.

### [Gel sheet]

The gel sheet 20 has a property of transmitting ultrasonic waves more in comparison to when a gel sheet is not used. The gel sheet 20 is a gel-like substance with silicone, for example, as a main ingredient, has a property of transmitting ultrasonic waves as well as having a suitable adhesive force, and can be disposed between the ultrasonic probe 12 and the subject M.

Also, the gel sheet 20 can be used repeatedly because its adhesive force does not decrease even when its front surface is washed with alcohol, etc.

Here, the gel sheet 20 does not necessarily have to be arranged with silicone as a main agent and may be arranged from any material as long as it transmits ultrasonic waves and can be adhered along the shape of the subject M.

FIG. 2 is an external view of the gel sheet 20 according to the first embodiment. The gel sheet 20 can be arranged in the form of a sheet. For example, a formed portion 21 that is formed in a predetermined region of the front surface to be more highly projecting and thereby made to differ in movement resistance in comparison to other regions and a positioning portion 22 for placing the gel sheet 20 on the subject M are included.

Here, the movement resistance refers to an ease of movement of the ultrasonic probe by a user when the ultrasonic probe 12 is moved over the front surface of the gel sheet 20 and can include, for example, frictional resistance.

Also, the movement resistance of the formed portion 21 may be made greater than or may be made less than a movement resistance of the other regions and it suffices that the formed portion 21 and the other regions differ relatively in movement resistance.

The positioning portion 22 can be made a penetrating hole of circular shape that is formed from the front surface to a rear surface of the gel sheet 20. And when placing the gel sheet 20 on the skin of the subject M, the gel sheet 20 can be placed easily at a predetermined position corresponding to a measurement site by aligning the positioning portion 22 with a part (for example, the navel, an elbow joint, a knee joint, a nipple, etc.) serving as a landmark of the subject M to be measured.

Here, although the positioning portion 22 does not necessarily have to be included, by including the positioning portion 22, the placement at the measurement site can be performed easily as described above. Also, since the placement location of the gel sheet 20 can be determined uniquely, the formed portion 21 of the gel sheet 20 and the scanning position of the ultrasonic probe 12 can be made to correspond in positional relationship as shall be described below.

Also, the positioning portion 22 does not necessarily have to be a penetrating hole formed in the gel sheet 20 and may be of any form as long as the gel sheet 20 can be aligned with the predetermined measurement site of the subject M and, for example, a depression may be formed in the front surface or rear surface of the gel sheet 20 or a predetermined mark may be engraved in the front surface or the rear surface.

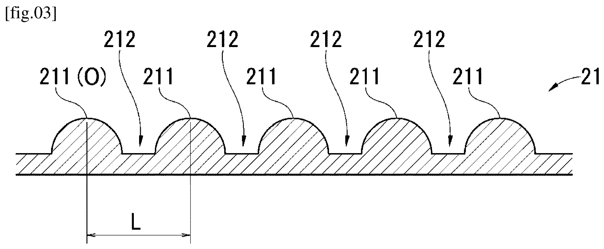

Next, the formed portion 21 of the gel sheet 20 shall be described. The region in which the formed portion 21 is formed is differed in movement resistance against the ultrasonic probe 12 in comparison to the other regions. For example, as shown in FIG. 3, in the formed portion 21, a plurality of projections 211 and recesses 212 of rectilinear shapes with a predetermined uniform width can be formed periodically continuously in correspondence to the scanning position of the ultrasonic probe 12. By forming a distance L between apexes of adjacent projections 211 at a fixed interval, for example, 1 mm, 1 cm, 5 cm, 10 cm, etc., the formed portion 21 can be made to function as a scale.

Also, the formed portion 21 and the other regions can be made to differ in movement resistance by an appropriate combination such as using a first material for the projections 211 and using a second material, differing in frictional force from the first material, for the recesses 212, etc.

Also, the formed portion 21 and the other regions can be made to differ in movement resistance, for example, by magnetizing the ultrasonic probe 12 and the formed portion 21.

Also, the formed portion 21 and the other regions can be made to differ in movement resistance, for example, by mounting a motor to the ultrasonic probe 12 and arranging such that the motor reacts electrically when the front surface of the formed portion 21 is scanned by the ultrasonic probe 12.

Here, the movement resistance can be made to differ within the region of the formed portion 21, for example, by making the projections 211 of the formed portion 21 be different instead of fixed in height.

In identifying the scanning position of the ultrasonic probe 12 in a state in which the gel sheet 20 is placed on the skin surface of the subject M, the user can count the number of scale marks from a starting point O of the projections 211 of the formed portion 21 to ascertain, based on a sensation due to the movement resistance against the ultrasonic probe 12, the measurement site specific to each subject M as a scale mark position without taking the eyes off the display portion 115.

Here, the user him/herself does not necessarily have to ascertain the scale mark position and, for example, a sensor that detects the movement resistance against the ultrasonic probe 12 can be included in the ultrasonic inspection system 1 and the scale mark position can be ascertained by detection by the sensor.

When the scale mark position is identified, the ultrasonic image of a scanning range can be obtained by scanning the ultrasonic probe 12 rectilinearly along the identified projection 211 based on the sensation due to the movement resistance against the ultrasonic probe 12 without taking the eyes off the display portion 115. Then, from the subsequent measurement onward, the ultrasonic probe 12 can easily be aligned with the correct scanning position each time by aligning the ultrasonic probe 12 with the scale mark position during the initial measurement (for example, the second peak from the projection 211 serving as the starting point O) based on the sensation due to the movement resistance against the ultrasonic probe 12 without taking the eyes off the display portion 115. Also, for example, even a user with poor eyesight can easily align the ultrasonic probe 12.

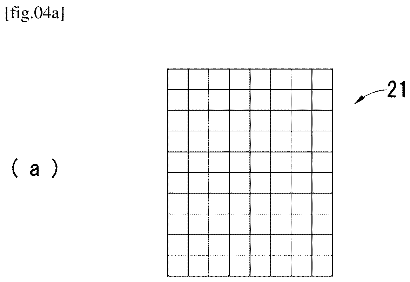

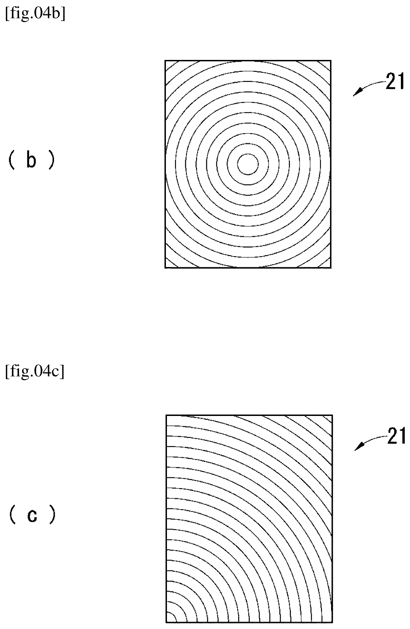

Here, the shape of the formed portion 21 does not necessarily have to be that in which the projections 211 and the recesses 212 of rectilinear shapes are formed continuously and, as shown in FIG. 4, various shapes such as a matrix shape (FIG. 4(a)), concentric circles (FIG. 4(b)), fan shapes (FIG. 4(c)), etc., can be adopted.

For example, the gel sheet 20 constituted of the formed portion 21 of matrix shape enables ascertainment of a pinpoint measurement position identified by a row position and a column position. This is thus effective when it is desired to identify a pinpoint measurement position rather than scanning the ultrasonic probe 12 in a predetermined range.

Also, when it is desired to perform a measurement by scanning the ultrasonic probe 12 in a scanning direction that is curved rather than rectilinear, the gel sheet 20 that includes the formed portion 21 constituted of shapes with curves, for example, concentric circles or fan shapes can be adopted. Thereby, once the scale mark position from the starting point O of the projections 211 of the formed portion 21 is identified, the intended ultrasonic image can be acquired by scanning the ultrasonic probe 12 along the projection corresponding to the scale mark.

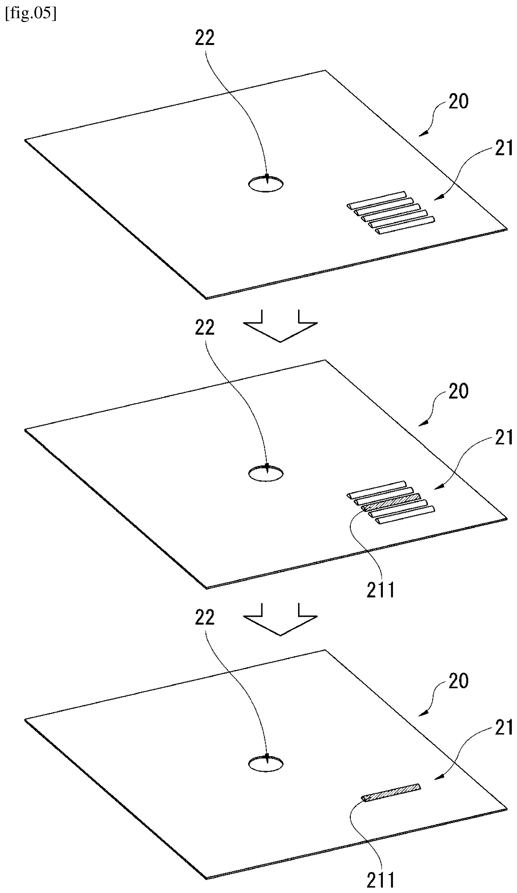

Also, when the scale mark of the scanning position of the ultrasonic probe 12 is determined in the initial measurement, it is possible to manufacture a gel sheet 20 that is customized based on the position information and perform measurements using the customized gel sheet 20 from the subsequent measurement onward. As a customization method for the gel sheet 20, manufacturing can be performed, for example, according to the procedure shown in FIG. 5.

### <STEP 1: Identifying the scanning position>

When the gel sheet 20 has been placed along the shape of the subject M, the user identifies the scanning position of the ultrasonic probe 12 while checking the ultrasonic image. As described above, the scanning position of the ultrasonic probe 12 is identified by the scale mark position from the starting point O of the projections 211 of the formed portion 21. Here, since the formed portion 21 and the other regions are differed in movement resistance, the user can easily identify the scanning position of the ultrasonic probe 12.

### <STEP 2: Marking of the scale mark>

Once the scanning position is identified in STEP 1, the projection 211 corresponding to the scanning position is marked. Here, the marking may be a marking on the gel sheet 20 or a marking on the system 1.

### <STEP 3: Customization of the gel sheet>

A second gel sheet 20 is prepared and, on its front surface, a projection 211 is formed at a position corresponding to the projection 211 marked in STEP 2. Here, the second gel sheet 20 may differ from the gel sheet used up to STEP 2 or the same gel sheet may be used.

By the above steps, the intended ultrasonic image can be acquired using the customized gel sheet 20 in measurements of the second time onward to align the ultrasonic probe 12 with the formed portion 21 formed on the front surface of the gel sheet 20 and simply scanning the ultrasonic probe 12 along the formed portion 21. Therefore, even a user who is unaccustomed to operation of the ultrasonic inspection device 10 can easily perform alignment of the ultrasonic probe 12.

Also, although in the above description, the projections 211 and the recesses 212 or combinations of the projections 211 and the recesses 212 constituting the formed portion 21 were described as including a mode of being formed continuously at uniform width, for example, the intervals of the projections 211 and the recesses 212 are not limited to a uniform width. For example, it is possible to set as appropriate within a single gel sheet or set as appropriate according to each lot of gel sheets or according to each user. Alternatively, heights of adjacent projections 211 may be arranged such as to differ.

Also, the formed portion 21 suffices to be such that when the ultrasonic probe 12 is scanned over the front surface of the gel sheet 20, respectively different movement resistances, for example, frictional forces are imparted in the region of the formed portion 21 and in the regions besides it, and instead of an uneven shape, the formed portion 21 and the regions besides it can be arranged from different materials. Here, when an uneven shape is not adopted as the formed portion 21, a scale for identifying the scanning position of the ultrasonic probe 12 can be indicated at the position corresponding to the formed portion 21. Also, an appropriate combination can be arranged such as using a first material for the projections 211 and using a second material differing in frictional force from the first material for the recesses 212.

Next, a second embodiment shall be described. In the following description, description of arrangements that are redundant to those of the first embodiment shall be omitted.

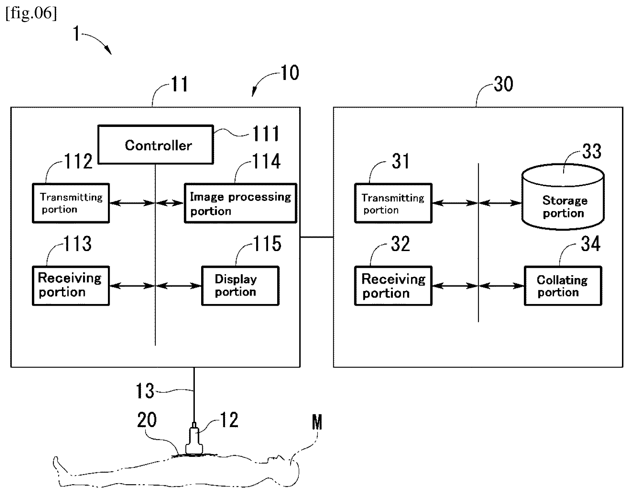

FIG. 6 is a schematic view of the ultrasonic inspection system 1 according to the second embodiment. In addition to the ultrasonic inspection device 10 and the gel sheet 20 that are arrangements of the first embodiment, the ultrasonic inspection system according to the second embodiment further includes a management server 30.

### [Gel sheet]

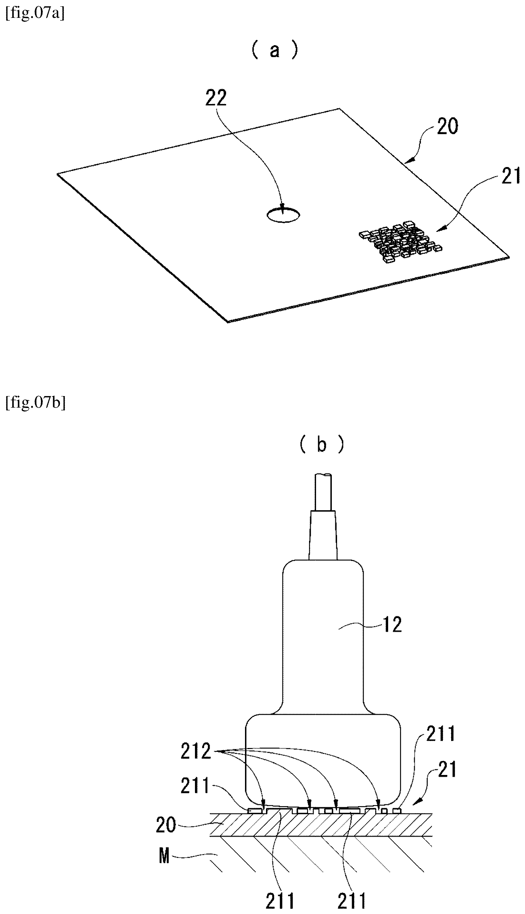

As shown in the plan view of FIG. 7(a), the formed portion 21 of the gel sheet 20 according to the second embodiment has an arrangement in which a plurality of projections 211 and recesses 212 are scattered across a predetermined range. These projections 211 and recesses 212 are formed in correspondence to predetermined digital information related to the subject M (for example, height, weight, sex, age, medical history, inspection history, and setting conditions of the ultrasonic probe 12).

Specifically, as shown in the sectional view of FIG. 7(b), when the formed portion 21 is scanned with the ultrasonic probe 12, whereas the projections 211 and the ultrasonic probe 12 are put in contact, the recesses 212 and the ultrasonic probe 12 are in a non-contacting state. In this state, since air layers are interposed between the ultrasonic probe 12 and the recesses 212, these become non-transmitting regions in which the ultrasonic waves transmitted from ultrasonic probe 12 cannot be transmitted through the gel sheet 20. That is, the recesses 212 of the formed portion 21 become the non-transmitting regions and the projections 211 of the formed portion 21 become transmitting regions.

Using the transmitting regions and the non-transmitting regions formed by the projections 211 and the recesses 212 and defining, for example, each non-transmitting region to be "0" as digital information and each transmitting region to be "1" as digital information, the formed portion 21 can be deemed to be predetermined digital information constituted of "0"s and "1"s.

By then scanning the formed portion 21 with the ultrasonic probe 12, the digital information is read and, by the ultrasonic inspection device 10, the read digital information is converted to electronic data and thereafter transmitted to the management server 30. Also, the gel sheet 20 according to the second embodiment can also be customized and manufactured based, for example, on the process of STEP 1 to STEP 3 illustrated with the first embodiment.

### [Management server]

The management server 30 is constituted of a transmitting portion 31, a receiving portion 32, a storage portion 33, and a collating portion 34 and is connected to the ultrasonic inspection device 10 by a communication means such as an internet line, etc. The management server 30 has functions such that when electronic data transmitted from the exterior are received by the receiving portion 32, the received electronic data are collated with predetermined electronic data stored in advance in the storage portion 33 and the matching electronic data are read out from the storage portion 33 and transmitted to the exterior via the transmitting portion 31.

The storage portion 33 is, for example, a ROM and a rewritable flash memory as internal memories and stores various electronic data such as height, weight, sex, age, medical history, inspection history, or setting conditions of the ultrasonic probe 12, etc., as information on each subject M in linkage with an ID. Registration of new subject information at any time is also enabled.

And when the formed portion 21 of the gel sheet 20 is scanned with the ultrasonic probe 12, the digital information formed in correspondence to the formed portion 21 is read by the ultrasonic probe 12 and converted to electronic data. The electronic data are received by the receiving portion 32 of the management server 30, the received electronic data and the electronic data stored in the storage portion 33 are further collated in the collating portion 34, and the matching electronic data are read out from the storage portion 33.

The electronic data read out from the storage portion 33 are transmitted from the transmitting portion 31 to the ultrasonic inspection device 10. At the ultrasonic inspection device 10, when the electronic data from the management server 30 are received, the contents of the electronic data are displayed on the display portion 115 and further, the ultrasonic probe 12 is automatically set to the setting conditions included in the electronic data.

Since by the above, the user can confirm that the information of the gel sheet 20 and the subject M are matched before measurement by the ultrasonic probe 12 using the gel sheet 20 and can thereupon perform the measurement by the ultrasonic probe 12, for example, mixing up of the subject can be prevented in advance. Since the ultrasonic probe 12 can be set automatically to the setting conditions for the subject M, there is no need to set the ultrasonic probe 12 each time and measurement can be performed simply and quickly.

Here, although the formed portion 21 of the gel sheet 20 according to the second embodiment is formed in correspondence to predetermined digital information as described above, the function of identifying the measurement position of the ultrasonic probe 12 may also be included as in the first embodiment by marking a portion of the projections 211 constituting the formed portion 21 or forming a second formed portion in a region different from the formed portion 21.

Also, in regard to the respective functions of the management server 30 according to the second embodiment, for example, the storage portion 33 and the collating portion 34 can be installed inside the ultrasonic inspection device 10 or can be arranged inside a memory of a user's smartphone or can be arranged on a virtual server such as a cloud server. Thereby, a need to provide a separate management server 30 is eliminated.

Although the first embodiment and the second embodiment have been described above, the first embodiment and the second embodiment can be combined as appropriate, for example, as in performing alignment easily with the first embodiment and reading digital information with the second embodiment, etc.

As described above, the gel sheet, the ultrasonic inspection system, and the method for manufacturing the gel sheet according to the present invention enable positioning and setting of the ultrasonic probe to be performed easily.

### Reference Signs List

1: ultrasonic inspection system
10: ultrasonic inspection device
11: ultrasonic inspection device main body

111: controller
112: transmitting portion
113: receiving portion
114: image processing portion
115: display portion
   12: ultrasonic probe
   13: cable
   20: gel sheet
   21: formed portion
211: projection
212: recess
   22: positioning portion
   30: management server
   31: transmitting portion
   32: receiving portion
   33: storage portion
   34: collating portion
   M: subject

## Claims

1. A gel sheet used by being interposed between an ultrasonic probe and a subject, wherein
a formed portion to which a treatment including any one of a projection, a recess, a periodically formed unevenness portion, or a predetermined surface treatment is applied in correspondence to predetermined information is included in a predetermined region of a front surface of the gel sheet and
the region in which the formed portion is formed differs in movement resistance against the ultrasonic probe in comparison to other regions.

2. The gel sheet according to Claim 1,
wherein the formed portion is formed in a region corresponding to a scanning location of the ultrasonic probe.

3. The gel sheet according to Claim 1 or Claim 2,
wherein the formed portion is formed in correspondence to the predetermined information related to the subject.

4. The gel sheet according to Claim 1 or Claim 2,
wherein the formed portion is formed in correspondence to a setting condition of the ultrasonic probe to be applied to the subject.

5. The gel sheet according to Claim 1 or Claim 2,
wherein the formed portion has a non-transmitting region that does not transmit the ultrasonic waves emitted from the ultrasonic probe and
the non-transmitting region is an air gap that is formed between the ultrasonic probe and the front surface of the gel sheet.

6. The gel sheet according to Claim 1 or Claim 2,
wherein the formed portion is formed periodically in a rectilinear shape, a matrix shape, or an arcuate shape formed along a displacement direction of the ultrasonic probe in plan view of the gel sheet.

7. An ultrasonic inspection system comprising:
an ultrasonic inspection device that has an ultrasonic probe having a sensor portion detecting reflected waves of emitted ultrasonic waves reflected from a subject and an image converting portion converting an electric signal from the ultrasonic probe to ultrasonic image data;
a gel sheet that is used by being interposed between the ultrasonic probe and the subject and has, in a predetermined region of a front surface, a formed portion to which a treatment including any one of a projection, a recess, a periodically formed unevenness portion, or a predetermined surface treatment is applied in correspondence to predetermined information, with the region in which the formed portion is formed being different in movement resistance against the ultrasonic probe in comparison to other regions; and
a management server that has a storage portion storing the predetermined information corresponding to the formed portion and a collating portion collating, by aligning the ultrasonic probe with the formed portion, the information stored in the storage portion and the information corresponding to the formed portion and is capable of selecting, based on a collation result of the collating portion, desired information from the information stored in the storage portion.

8. The ultrasonic inspection system according to Claim 7,
wherein the information corresponding to the formed portion is a setting condition of the ultrasonic probe to be applied according to each subject.

9. The ultrasonic inspection system according to Claim 7 or Claim 8,
wherein the information corresponding to the formed portion is specific information of the subject including a height, weight, sex, age, medical history, and inspection history of each subject.

10. A method for manufacturing a gel sheet comprising:
a step of marking a mark indicating a positional relationship of an ultrasonic probe and a gel sheet on the gel sheet in a state in which the gel sheet is interposed between the ultrasonic probe and a subject; and
a step of forming, in a predetermined region that includes a position corresponding to the mark marked on the gel sheet, a formed portion differing in movement resistance against the ultrasonic probe in comparison to other regions.
